# EUROPEAN PATENT APPLICATION

(11) **EP 2 921 106 A1**
(43) Date of publication of application: **23.09.2015**
(21) Application number: 15159420.7
(22) Date of filing: 17.03.2015
(51) Int. Cl.: A61B 5/024

(54) **PORTABLE ELECTRONIC DEVICE AND METHOD FOR PHYSIOLOGICAL MEASUREMENT**

(30) Priority: 17.03.2014 US 201461953942 P; 09.12.2014 US 201414564098
(71) Applicant: HTC Corporation, Taoyuan City 330 (TW)
(72) Inventor: Liang, Yu-Hsin, 330, Taoyuan District, Taoyuan City (TW); Wu, Heng-Chien, 330, Taoyuan District, Taoyuan City (TW); Chu, Shih-Hung, 330, Taoyuan District, Taoyuan City (TW)
(74) Representative: Emde, Eric

(57) **Abstract**

A method for physiological measurement and a portable electronic device adapted for executing the method. The portable electronic device includes a button and a display. The method for physiological measurement includes the following steps: transmitting at least one trigger signal when the button is pressed; transmitting a first optical signal towards the outside of the button through the button according to the trigger signal; receiving a second optical signal through the button; and obtaining physiological information according to the second optical signal, in which the second optical signal is an optical signal that is reflected when the first optical signal encounters an object.

## Description

### CROSS-REFERENCE TO RELATED APPLICATION

This application claims the priority benefits of U.S. provisional application serial no. 61/953,942, filed on March 17, 2014. The entirety of the above-mentioned patent application is hereby incorporated by reference herein and made a part of this specification.

### BACKGROUND OF THE INVENTION

### Field of the Invention

The invention is related generally to an electronic device having a measurement function, and more particularly to a portable electronic device and a method for physiological measurement.

### Description of Related Art

Current portable electronic devices have a remote control function, and these devices can serve as a typical consumer electronics remote control that transmits infrared signals to control the home electronic equipment, such as for changing the television channels, or adjusting the television volume. Some portable electronic devices may transmit infrared signals to the fingers of a user to use the reflection signal on the user's fingers to measure the heart beat rate of the user.

Although both uses infrared signals, in current portable electronic devices, the remote control function and the heart beat measurement function are not integrated in one hardware module but implemented by two different hardware modules. In order to transmit and receive signals, two holes need to be configured on the housing of the portable electronic device, which adds complexity to the housing design. Moreover, heart rate measurement requires complex operations. The user must wake the mobile phone, unlock the touch screen, start the corresponding application software, and place a finger on the measurement hole of the housing. These issues require technological improvements.

### SUMMARY OF THE INVENTION

The invention provides a portable electronic device and a method for physiological measurement, for mitigating the afore-described issues of the conventional portable electronic device.

The invention provides a portable electronic device including a sensing module and a transceiver circuit. The sensing module includes a button, a transmitter unit, and a receiver unit. The sensing module transmits at least one trigger signal when the button is pressed. The transmitter unit is disposed inside the button, and the transmitter unit is configured to transmit a first optical signal towards the outside of the button through the button in response to the trigger signal. The receiver unit is disposed inside the button, and the receiver unit receives a second optical signal through the button. Moreover, the second optical signal is an optical signal that is reflected when the first optical signal encounters an object. The transceiver circuit is coupled to the sensing module, and the transceiver circuit is configured to control the transmitter unit to transmit the first optical signal towards the outside of the button through the button. Furthermore, the transceiver circuit obtains physiological information according to the second optical signal. The button has a transparent material to allow the first optical signal and the second optical signal to travel through the button.

The invention provides a method for physiological measurement executed by a portable electronic device. The portable electronic device includes a button and a display. The method for physiological measurement includes the following steps: transmitting at least one trigger signal when the button is pressed, transmitting a first optical signal towards the outside of the button through the button according to the trigger signal, receiving a second optical signal through the button, and obtaining physiological information according to the second optical signal. Moreover, the second optical signal is an optical signal that is reflected when the first optical signal encounters an object.

According to embodiments of the invention, the portable electronic device and the method for physiological measurement may adopt a single sensing module to measure the physiological information of the user, thereby not only reducing the housing holes of the portable electronic device, but also drastically simplifying the operation process of measuring the physiological information.

### BRIEF DESCRIPTION OF THE DRAWINGS

FIG. 1 is a schematic view of a portable electronic device 100 according to an embodiment of the invention.
FIG. 2A-FIG. 2E are schematic views of a sensing module of a portable electronic device according to a plurality of embodiments of the invention.
FIG. 3A-FIG. 3D are schematic views of a transceiver circuit of a portable electronic device according to a plurality of embodiments of the invention.
FIG. 4 and FIG. 5 are flow diagrams of a method for physiological measurement according to an embodiment of the invention.
FIG. 6 is a schematic view of a display image of a portable electronic device according to an embodiment of the invention.
FIG. 7 is a schematic view of a display image of a portable electronic device according to another embodiment of the invention.

### DESCRIPTION OF EMBODIMENTS

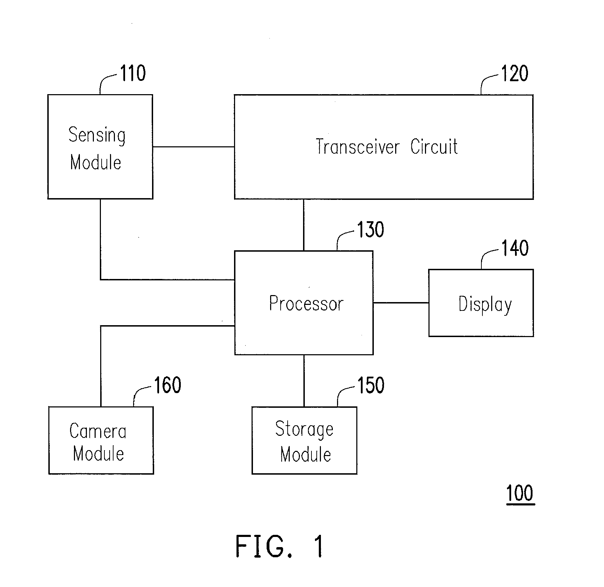

FIG. 1 is a schematic view of a portable electronic device 100 according to an embodiment of the invention. The portable electronic device 100 may be a smartphone, a personal digital assistant (PDA), a tablet computer, or wearable electronic devices manufactured to have a watch appearance, for example. The portable electronic device 100 includes a sensing module 110, a transceiver circuit 120, a processor 130, a display 140, a storage device 150, and a camera module 160. The transceiver circuit 120 is coupled to the sensing module 110, and the processor 130 is coupled to the sensing module 110 and the transceiver circuit 120. The display 140, the storage device 150, and the camera module 160 are all coupled to the processor 130.

The sensing module may transmit optical signals to remotely control other electronic devices. The sensing module 110 may also transmit optical signals to a finger of the user or other skin parts, receive the optical signals reflected from the user's finger or the other skin parts, and convert the optical signals into electric signals for measuring physiological information of the user. The physiological information may be, for example, a heart beat rate, a heart waveform, a blood oxygenation level, or a combination of the foregoing information. The transceiver circuit may provide the signal transmitted by the sensing module 110 and analyze the electric signal converted by the sensing module 110, so as to obtain the physiological information of the user. The processor 130 may execute an operating system and application software of the portable electronic device 100. The display 140 is a touch display capable of displaying a user interface of the portable electronic device 100 and receiving a touch input of the user. The storage device 150 may be a memory or a hard drive. Moreover, the storage device 150 may store the physiological information of the user, as well as the operating system, application software, and operation data of the portable electronic device 100. The camera module 160 may capture an image. After the image is processed by the processor 130, the image may be stored in the storage device 150.

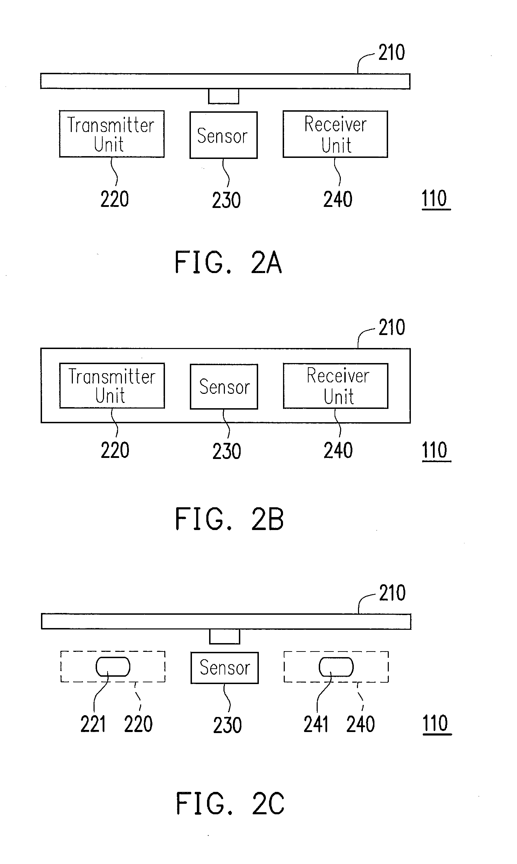

FIG. 2A is a schematic side view of the sensing module 110 of the portable electronic device 100 according to an embodiment of the invention. The sensing module 110 includes a button 210, a transmitter unit 220, a sensor 230, and a receiver unit 240. The button 210 is disposed on a housing of the portable electronic device 100, and the transmitter unit 220, the sensor 230, and the receiver unit 240 are disposed inside the button 210. The outside of the button 210 is defined as a top portion of FIG. 2A, which is also an outward direction of the portable electronic device 100. The inside of the button 210 is defined as a lower portion of FIG. 2A, which is also an inward direction of the portable electronic device 100. The sensor 230 is coupled to the transceiver circuit 120 and the processor 130. The transmitter unit 220 and the receiver unit 240 are coupled to the transceiver circuit 120.

When the sensor 230 senses that the button 210 is pressed, the sensor 230 transmits a trigger signal to the transceiver circuit 120 and the processor 130. When the transceiver 120 receives the trigger signal, the transmitter unit 220 is controlled to transmit an optical signal to execute the afore-described measurement function. The optical signal may be an infrared signal, a red light signal, or both. The receiver unit 240 may receive the optical signal for the measurement function. The button 210 has a transparent material to allow the infrared and red light to travel through. The transmitter unit 220 transmits the optical signal through the button 210. That is, the optical signal transmitted by the transmitter unit 220 is transmitted towards the outside of the portable electronic device 100 through the button 210. Moreover, the receiver unit 240 receives optical signals through the button 210. That is, optical signals outside of the portable electronic device 100 enter inside the portable electronic device 100 through the button 210 to be received by the receiver unit 240. The transmitter unit 220 includes at least one optical transmitter to transmit optical signals, and the receiver unit 240 includes at least one optical receiver to receive optical signals. The optical transmitter may be implemented by an infrared or red light-emitting diode (LED), and the optical receiver may be implemented by a photodiode.

FIG. 2B is a side schematic view of the sensing module 110 of FIG. 2A. In this viewing angle, the transmitter unit 220, the sensor 230, and the receiver unit 240 and disposed below the button 210. An area of the button 210 is large enough to cover the transmitter unit 220, the sensor 230, and the receiver unit 240. In the present embodiment, the button 210 may be a start button of the portable electronic device 100. When the user presses the button 210 to start or wake up the portable electronic device 100, if a finger of the user remains on the button 210, then the portable electronic device 100 may measure the physiological measurement of the user at this time.

FIG. 2C is a side schematic view of the sensing module 110 according to an embodiment of the invention. In the present embodiment, the transmitter unit 220 includes an optical transmitter 221, the receiver unit 240 includes an optical receiver 241. The optical transmitter 221 and the optical receiver 241 are disposed inside the button 210 and coupled to the transceiver circuit 120. The sensing module 110 of the present embodiment may be configured for remotely controlling other electronic devices. The optical transmitter 221 may be controlled by the transceiver circuit 120 to transmit an infrared signal towards the outside of the button 210 through the button 210, so as to remotely control another electronic device, such as a television. The optical receiver 241 may receive an infrared signal from a certain electronic device through the button 210, such as the infrared signal transmitted by a remote control. The transceiver circuit 120 may learn a remote control signal of a remote control according to the infrared signal.

In another embodiment, the sensing module 110 of FIG. 2C may measure a heart beat rate or a heart waveform of the user. Infrared or red light may be configured for these heart beat measurements. Accordingly, the optical transmitter 221 may be controlled by the transceiver circuit 120 to transmit an infrared signal or a red light signal through the button 210. Moreover, the optical receiver 241 may correspondingly receive the infrared signal or red light signal reflected from the finger of the user through the button 210.

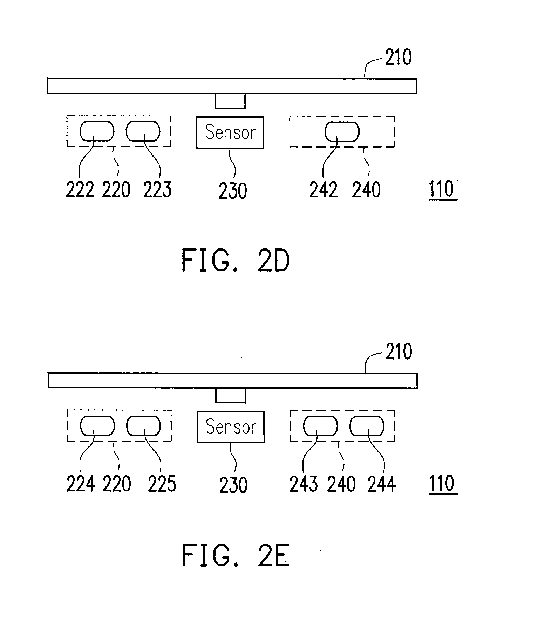

FIG. 2D is a side schematic view of the sensing module 110 according to an embodiment of the invention. In the present embodiment, the transmitter unit 220 includes two optical transmitters 222 and 223, and the receiver unit 240 includes an optical receiver 242. The optical transmitters 222 and 223 and the optical receiver 242 are disposed inside the button 210 and coupled to the transceiver circuit 120. The optical transmitter 222 may be controlled by the transceiver circuit 120 to emit the infrared signal through the button 210, and the optical transmitter 223 may be controlled by the transceiver circuit 120 to emit the red light signal through the button 210. The optical receiver 242 may receive the reflected infrared signal and red light signal through the button 210. Moreover, the optical receiver 242 may also receive infrared signals from other electronic devices, such as an infrared signal from a remote control. The sensing module 110 of the present embodiment may also be configured for remotely controlling other electronic devices, measuring the heart beat rate and heart waveform of the user, and measuring the blood oxygenation level of the user. Infrared light or red light may be used to measure the heart beat rate and heart waveform. If the blood oxygenation level needs to be measured, then both the infrared and red light are required.

FIG. 2E is a side schematic view of the sensing module 110 according to another embodiment of the invention. In the present embodiment, the transmitter unit 220 includes two optical transmitters 224 and 225, and the receiver unit 240 includes two optical receivers 243 and 244. The optical transmitters 224 and 225 and the optical receivers 243 and 244 are disposed inside the button 210 and coupled to the transceiver circuit 120. The optical transmitter 224 may be controlled by the transceiver circuit 120 to emit the infrared signal through the button 210, and the optical transmitter 225 may be controlled by the transceiver circuit 120 to emit the red light signal through the button 210. The optical receiver 243 may receive the reflected infrared signal from the remote control through the button 210, and the optical receiver 244 may receive the reflected red light signal through the button 210. The sensing module 110 of the present embodiment may also be configured for remotely controlling other electronic devices, measuring the heart beat rate and heart waveform of the user, and measuring the blood oxygenation level of the user.

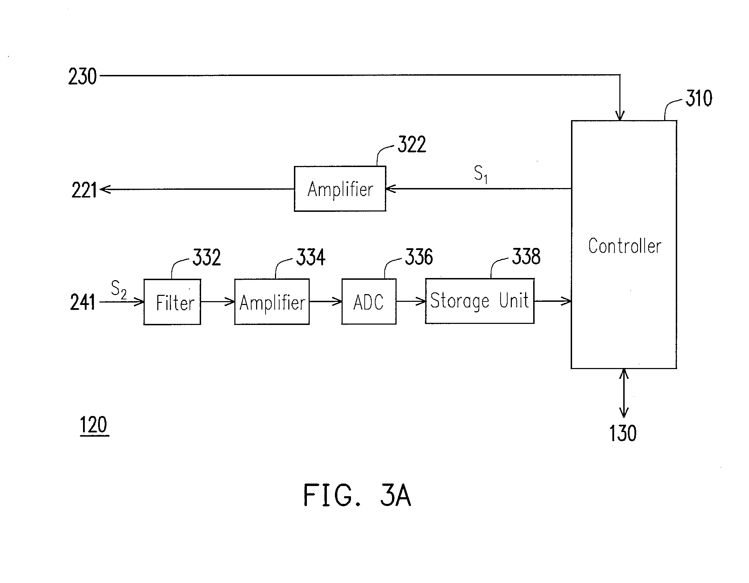

FIG. 3A is a schematic view of the transceiver circuit 120 of the portable electronic device 100 according to an embodiment of the invention. The transceiver circuit 120 of the present embodiment is coupled to the sensing module 110 of FIG. 2C, and the transceiver circuit 120 includes a controller 310, an amplifier 322, a filter 332, an amplifier 334, an analog-to-digital converter (ADC) 336, and a storage unit 338. The amplifier 322 is coupled between the optical transmitter 221 and the controller 310. The filter 332, the amplifier 334, the ADC 336, and the storage unit 338 are serially coupled between the optical transmitter 241 and the controller 310 in sequence. The controller 310 is also coupled to the sensor 230 and the processor 130.

When the user presses the button 210, the controller 310 receives the trigger signal from the sensor 230, and then the controller 310 transmits an electric signal S₁ in response to the trigger signal. The amplifier 332 amplifies the electric signal S₁ and outputs to the optical transmitter 221. The optical transmitter 221 converts the amplified electric signal S₁ into an optical signal and the optical signal is transmitted through the button 210. When the optical signal transmitted by the optical transmitter 221 encounters an object, such as the finger of the user or other skin parts, the optical signal is reflected back and travels through the button 210 to be received by the optical receiver 241. Thereafter, the optical receiver 241 converts the reflected optical signal into another electric signal S₂ and sends the electric signal S₂ to the filter 332. The filter 332 filters out a noise signal in the electric signal S₂, and the amplifier 334 amplifies the electric signal S₂. The ADC 336 converts the amplified electric signal S₂ from an analog signal into a digital signal. The storage unit 338 converts the digital signal outputted by the ADC 336 into a binary value, and the storage unit 338 stores this binary value corresponding to the digital value for access by the controller 310. After the controller 310 collects and analyzes this binary value, the physiological information of the user can be obtained according to the binary value.

In the present embodiment, the controller 310 may use the electric signal S₁ to control the optical transmitter 221 to transmit the infrared signal or the red light signal, and the controller 310 may analyze the values in the storage unit 338 to obtain the heart beat rate and/or the heart waveform of the user. The techniques for analyzing signals to obtain the heart beat rate and heart waveform are conventionally known in the art, and therefore further elaboration thereof is omitted hereafter.

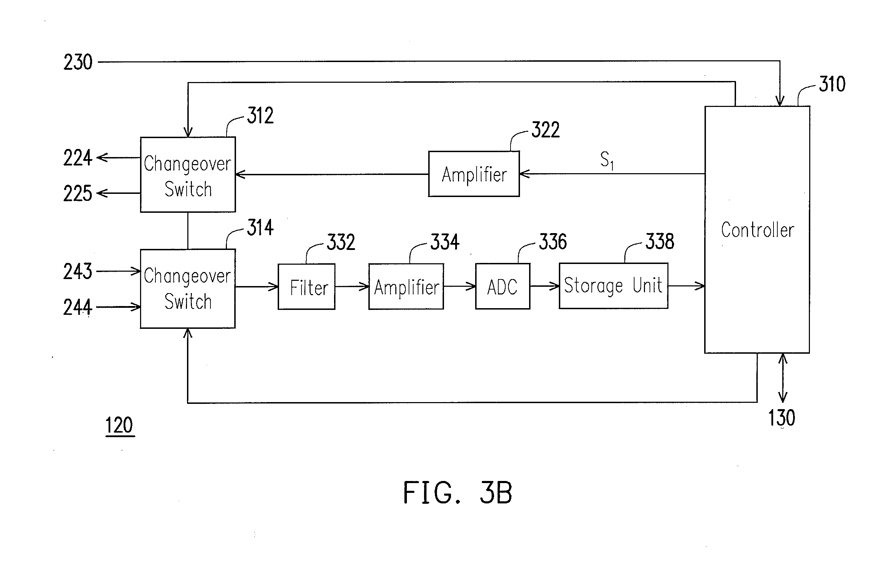

FIG. 3B is a schematic view of the transceiver circuit 120 according to another embodiment of the invention. The transceiver circuit 120 of the present embodiment is coupled to the sensing module 110 of FIG. 2E. A difference between the transceiver circuit 120 of FIG. 3B and the transceiver circuit 120 of FIG. 3A is in the two additional changeover switches 312 and 314. When infrared signals need to be transmitted and received, the controller 310 controls the changeover switch 312 to connect to the amplifier 322 and the optical transmitter 224, and controls the changeover switch 314 to connect to the optical receiver 243 and the filter 332. When red light signals need to be transmitted and received, the controller 310 controls the changeover switch 312 to connect to the amplifier 322 and the optical transmitter 225, and controls the changeover switch to connect to the optical receiver 244 and the filter 332.

Similar to the transceiver circuit 120 of FIG. 3A, the transceiver circuit 120 of FIG. 3B may be configured for measuring the heart beat rate and/or the heart waveform of the user. Moreover, the transceiver circuit 120 of FIG. 3B may be configured for measuring the blood oxygenation level of the user. The controller 310 may transmit the electric signal S₁ to control the optical transmitter 24 to transmit the infrared signal, analyze the values in the storage unit 338, transmit the electric signal S₁ again to control the optical transmitter 225 to transmit the red light signal, analyze the values in the storage unit 338 again, so as to obtain the blood oxygenation level of the user. The techniques for analyzing signals to obtain the blood oxygenation level are conventionally known in the art, and therefore further elaboration thereof is omitted hereafter.

In another embodiment, the transceiver circuit 120 of FIG. 3B may be simplified to couple to the sensing module 110 of FIG. 2D. In the present embodiment, the changeover switch 312 may be coupled to the amplifier 322 and the optical transmitters 222 and 223. The changeover switch 314 may be omitted. The filter 332 may be coupled to the optical receiver 242.

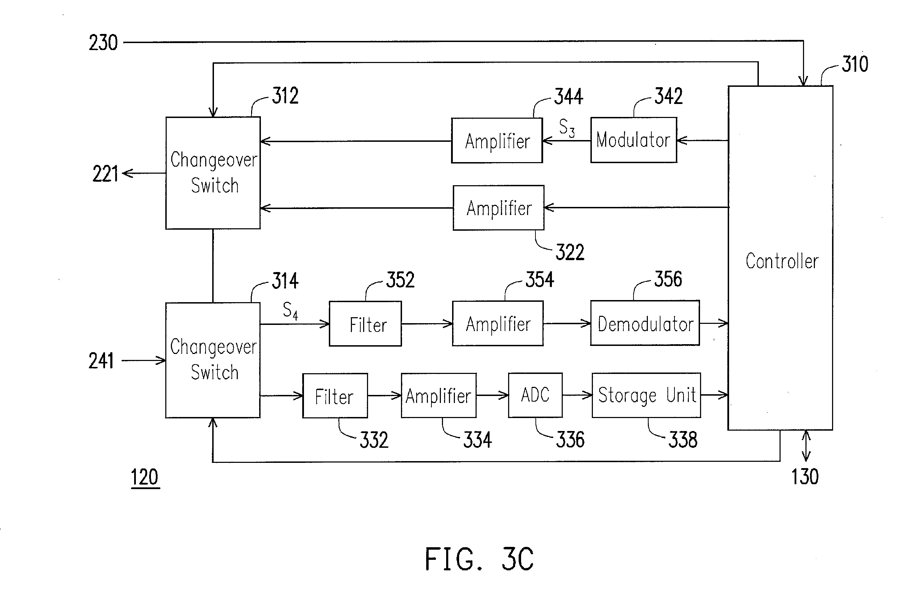

FIG. 3C is a schematic view of the transceiver circuit 120 according to another embodiment of the invention. The transceiver circuit 120 of the present embodiment is coupled to the sensing module 110 of FIG. 2C. A difference between the transceiver circuit 120 of FIG. 3C and the transceiver circuit of FIG. 3A is in the two additional changeover switches 312 and 314, a modulator 342, an amplifier 344, a filter 352, an amplifier 354, and a demodulator 356. The modulator 342 and the amplifier 344 are serially coupled between the controller 310 and the changeover switch 312 in sequence. The filter 352, the amplifier 354, and the demodulator 356 are serially coupled between the changeover switch 314 and the controller 310 in sequence. Besides the changeover switches 312 and 314, the additional elements above are configured for a remote control function of the portable electronic device 100.

The transceiver circuit of FIG. 3C may be configured for remotely controlling other electronic devices or measuring the heart beat rate and/or the heart waveform of the user. When other electronic devices need to be remotely controlled, the controller 310 controls the changeover switch 312 to connect to the amplifier 344 and the optical transmitter 221, and controls the changeover switch 314 to connect to the optical receiver 241 and the filter 352. When the heart beat rate and/or the heart waveform of the user need to be measured, the controller 310 controls the changeover switch 312 to connect to the amplifier 322 and the optical transmitter 221, and controls the changeover switch 314 to connect to the optical receiver 241 and the filter 332.

When remotely controlling other electronic devices, the controller 310 transmits a remote control command. The modulator 342 modulates and generates an electric signal S₃ according to the remote control command. The amplifier 344 amplifies the electric signal S₃. The optical transmitter 221 converts the amplified electric signal S₃ into the infrared signal, and transmits this infrared signal towards the outside of the button 210 through the button 210, so as to remotely control another electronic device.

The transceiver circuit 120 of FIG. 3C may also learn the remote commands of other remote controls. The optical receiver 241 may receive an infrared signal from other remote controls through the button 210, and convert this infrared signal into another electric signal S₄. The filter 352 filters out a noise signal in the electric signal S₄. The amplifier 354 amplifies the electric signal S₄. The demodulator 356 demodulates the amplified electric signal S₄ to obtain a remote control command in the electric signal S₄. The controller 310 may learn this remote control command.

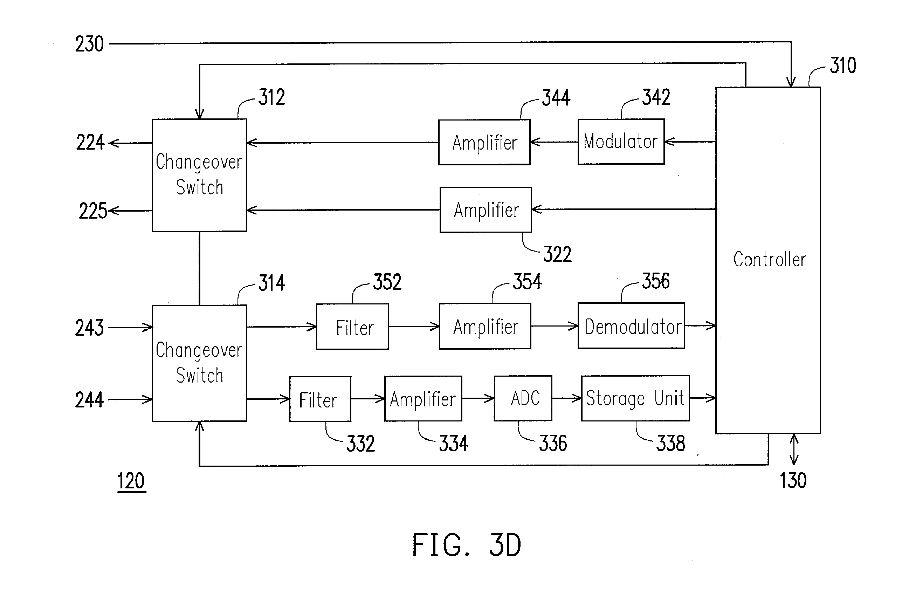

FIG. 3D is a schematic view of the transceiver circuit 120 according to another embodiment of the invention. The transceiver circuit 120 of the present embodiment is coupled to the sensing module 110 of FIG. 2E. A difference between the transceiver circuit 120 of FIG. 3D and the transceiver circuit 120 of FIG. 3C is that, the changeover switches 312 and 314 are coupled to the sensing module 110 of FIG. 2E. The controller 310 may control the changeover switch 312 to connect to the amplifier 344 and the optical transmitter 224, so as to transmit an infrared signal for the remote control function. Alternatively, the controller 310 may control the changeover switch 312 to connect to the amplifier 322 and the optical transmitter 224, so as to transmit an infrared signal for a physiological measurement function. Alternatively, the controller 310 may control the changeover switch 312 to connect to the amplifier 322 and the optical transmitter 225, so as to transmit a red light signal for a physiological measurement function. Moreover, the controller 310 may control the changeover switch 314 to connect to the optical receiver 243 and the filter 352, so as to receive the infrared signal for the remote control function. Alternatively, the controller 310 may control the changeover switch 314 to connect to the optical receiver 243 and the filter 332, so as to receive the infrared signal for the physiological measurement function. Alternatively, the controller 310 may control the changeover switch 314 to connect to the optical receiver 244 and the filter 332, so as to receive the red light signal for the physiological measurement function.

The transceiver circuit of FIG. 3D is capable of transmitting infrared signals and red light signals, as well as receiving infrared signals and red light signals. Therefore, the transceiver circuit of FIG. 3D may be configured to control another electronic device and to measure the heat beat rate, the heart waveform, and the blood oxygenation level of the user.

In another embodiment, the transceiver circuit 120 of FIG. 3D may be simplified to couple to the sensing module 110 of FIG. 2D. In the present embodiment, the changeover switch 312 may be coupled to the amplifiers 344 and 322 and the optical transmitters 222 and 223. The changeover switch 314 may be coupled to the optical receiver 242 and the filters 352 and 332.

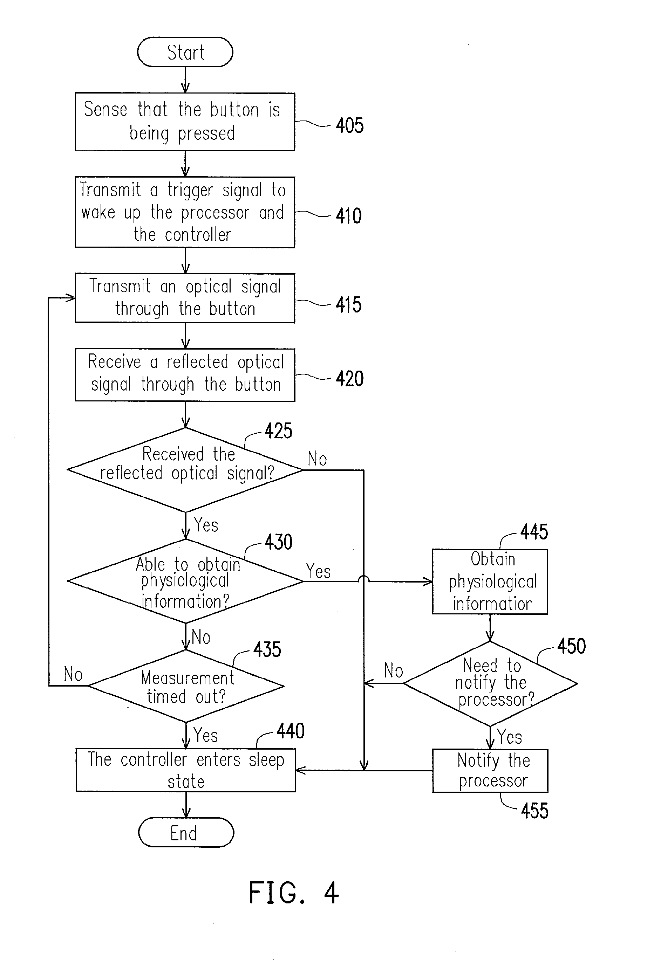

FIG. 4 is a flow diagram of a method for physiological measurement according to an embodiment of the invention. The portable electronic device 100 may execute this method to measure the physiological information of the user. When the user presses the button 210, the sensor 230 senses the button 210 is being pressed in step 405. In step 410, the sensor 230 transmits a trigger signal to wake up the processor 130, and transmits another trigger signal to wake up the controller 310. In another embodiment, the sensor 230 may transmit a same trigger signal to the processor 130 and the controller 310. When the processor 130 receives the trigger signal, the processor 130 may control the portable electronic device 100 to enter an operating state from a sleep state according to the trigger signal. In the present embodiment, the sleep state and the operating state of the portable electronic device 100 is defined as whether the display 140 is displaying an image. When the portable electronic device 100 is in the sleep state, the display 140 is turned off. On the other hand, when the portable electronic device 100 is in the operating state, the display 140 is turned on so as to display an operating image.

The controller 310 receives the trigger signal from the sensor 230, and in step 415, according to the trigger signal, the controller 310 controls the optical transmitter of the sensing module 110 to transmit a first optical signal towards the outside of the button 210 through the button 210. In Step 420, the optical receiver of the sensing module 110 then receives a second optical signal through the button 210 that is reflected back by an object when the transmitted first optical signal encounters the object. In step 425, the controller 310 checks whether the optical receiver has received the reflected second optical signal. When the reflected second optical signal is not received, this represents that the finger of the user has been immediately released after pressing the button 210, such that the physiological information cannot be measured, and therefore the controller 310 enters the sleep state in step S440.

When the optical receiver receives the reflected optical signal, in step 430, the controller 310 detects whether a value provided by the storage unit 338 is sufficient to obtain the physiological information of the user. If not sufficient, the controller 310 in step 435 checks whether the current physiological measurement has timed out. A time limit for this timeout may be set as 0.5 seconds, 1 second, 2 seconds, or 3 seconds, for example. If the current physiological measurement has not timed out, the process returns to step 415 to collect more values for the controller 310 to analyze. If the current physiological measurement has timed out, the controller 310 enters the sleep state in step 440.

Returning to step 430, when the detection result indicates that the value provided by the storage unit 338 is sufficient to obtain the physiological information of the user, then in step 445, the controller 310 analyzes the value to obtain the physiological information of the user. In step 450, the controller 310 checks whether it is necessary to notify the processor 130 to read the physiological information, since the physiological information may accumulate until a preset amount before the processor 130 needs to be notified to read the physiological information. When the processor 130 does not need to be notified, the controller 310 enters the sleep state in step 440. When the processor 130 needs to be notified, the controller 310 transmits a notification signal to the processor 130 in step 455. The processor 130 obtains the physiological information of the user from the controller 310 according to the notification signal. The processor 130 may store the physiological information in the storage device 150, control the display 140 to display the physiological information, or further process the physiological information.

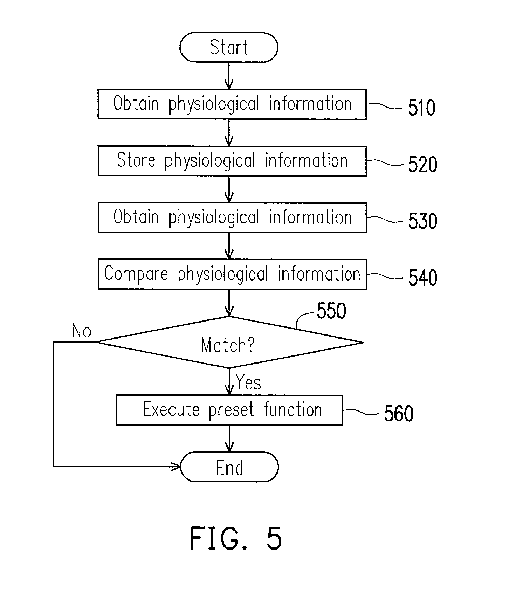

FIG. 5 is a flow diagram of a method for physiological measurement according to another embodiment of the invention. The portable electronic device 100 may execute this method to verify an identity of the user using the physiological information. For example, the heart waveform may used like a fingerprint to verify the identity. In step 510, the portable electronic device 100 may execute the method depicted in FIG. 4, so as to obtain the physiological information of a certain user. In step 520, the processor 130 may store the physiological information in the storage device 150. The physiological information may serve as an identity certificate of the user. In step 530, when the button 210 is pressed by the user, the portable electronic device 100 may execute the method depicted in FIG. 4 again, so as to obtain the physiological information of the user. In the present embodiment, the button 210 is the start button of the portable electronic device 100.

In step 540, the processor 130 may compare the physiological information measured in step 530 and the physiological information stored in the storage device 150. In Step 550, whether the physiological information obtained in the two measurements matches each other is determined. When the physiological information of the two measurements do not match, this represents that the user of step 530 is not the correct user, and the process ends. When the physiological information of the two measurements match each other, this represents that the user of step 530 is the correct user, and the processor 130 may execute a corresponding preset function in step 560.

The afore-described preset function may be a function that unlocks a locked state of the display 140. As described earlier, the display 140 is a touch display. In the present embodiment, the locked state refers to the display 140 displaying a lock screen, in which various conventional slide unlock or password unlock methods may be used to unlock the locked state and enter an operable state, so as to display various application program icons and function keys for touch operation by the user. However, by using the method depicted in FIG. 5, the portable electronic device 100 may use the physiological information to verify the identity of the user, and thereby directly unlock the locked state of the display 140 without resorting to the conventional slide unlock or password unlock methods. Alternatively, the preset function may further include controlling the display 140 to display the physiological information of the user. Alternatively, the preset function may further include controlling the display 140 to display a related message of the physiological information. For example, according to the heart beat rate of the user, the processor 130 may control the display 140 to display a recommendation message of an application software. For instance, when the heart beat rate of the user exceeds a preset threshold value, the processor 130 may recommend the user to execute a music playback software or a game software to calm the mood. The preset function may also be a combination of all of the foregoing functions or a part of the foregoing functions.

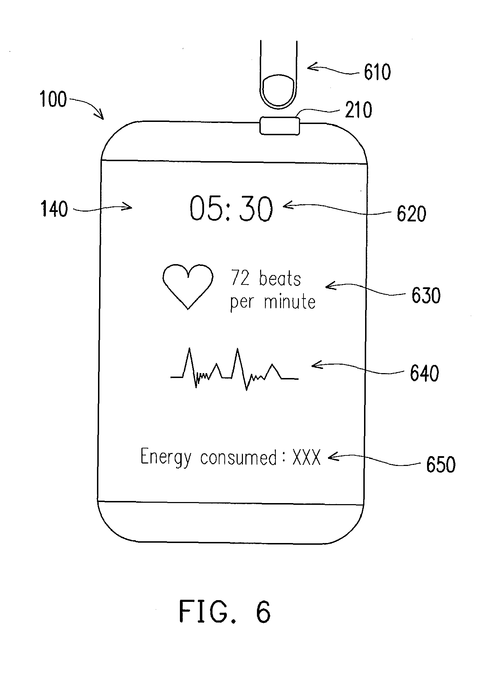

FIG. 6 is a schematic view of a display image of the display 140 of the portable electronic device 100 according to an embodiment of the invention. In the present embodiment, the button 210 is the start button of the portable electronic device 100. When the user uses a finger 610 to press the button 210, and after identity verification is completed through steps 530, 540, and 550 of FIG. 5, the preset function executed by the processor 130 may be controlling the display 140 to display an image shown in FIG. 6. In the present embodiment, the display image of the display 140 may include a time 620, a heart beat rate 630 of the user, a heart waveform 640 of the user, and an energy (calorie) consumption message 650 of the user. The time 620 may be the current time or the stopwatch time. The processor 130 may calculate the energy consumption according to the heart beat rate 630 of the user, in order to display the energy consumption message 650. The energy consumption message 650 may display the energy consumed in a unit time, or an accumulated energy consumed during the stopwatch time period.

In other embodiments, the steps 510, 520, 530, 540, and 550 of FIG. 5 may be omitted. After the user picks up the portable electronic device 100 and presses the button 210, the physiological information can be measured and the display image of FIG. 6 can be seen, with rapid response and convenience. Complex operations such as the conventional display unlock and application software execution are not needed. Moreover, the button 210 may be a power button that starts or wakes the portable electronic device 100. When the portable electronic device 100 is in the sleep state, the button 210 only needs to be pressed once to wake the portable electronic device 100, unlock the display 140, as well as measure the physiological information, thereby enhancing the convenience in viewing the physiological information or performing the subsequent operations.

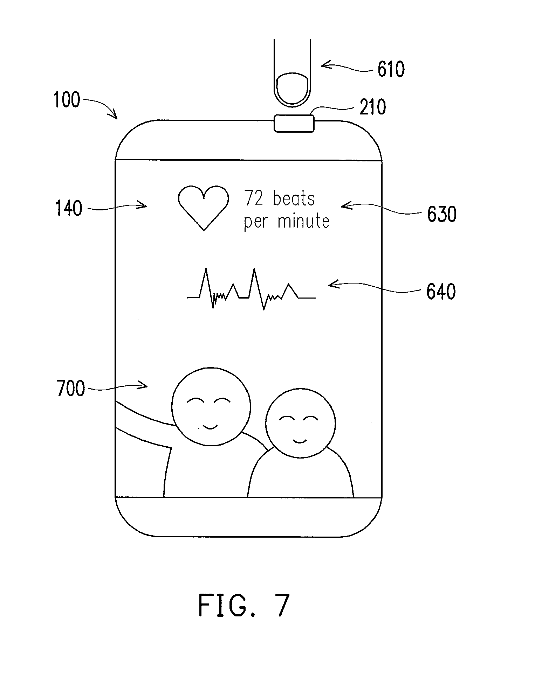

FIG. 7 is a schematic view of a display image of the display 140 of the portable electronic device 100 according to another embodiment of the invention. With reference to FIG. 1 and FIG. 7, in the present embodiment, the button 210 is a shutter button of the camera module 160 in the portable electronic device 100 configured for capturing photos. When a user 700 starts a camera self-photo function on the portable electronic device 100 and uses the finger 610 to press the button 210 to capture a photo through the camera module 160, the portable electronic device 100 may execute the method depicted in FIG. 4 to measure the physiological information of the user 700, such as the heart beat rate 630 and the heart waveform 640. Thereafter, the physiological information measured by the processor 130 may be edited and inserted on the captured photo by using image processing methods, and the edited photo may be stored in the storage device 150.

In summary, according to embodiments of the invention, the physiological information of the user may be measured while a function key (e.g. start button or camera shutter button) of the portable electronic device is pressed by the user, thereby saving the conventional complex operations. The simple and convenient method increases the inclination of the user to use this measurement function. The measured physiological information may be used to verify the user identity, as well as preset functions such as unlocking the display or displaying the physiological information. The display unlock mechanism only requires the user to press a button once, without requiring password entry or executing special operations. Embodiments of the invention adopt a single sensing module to execute the remote control function and the measurement function, thereby reducing hardware costs and space requirements. Moreover, the remote control and measurement functions employ the currently available start button or camera shutter button for transmitting and receiving optical signals. Therefore, embodiments of the invention do not require holes to be configured in the housing of the portable electronic device for the remote control and measurement functions, thereby simplifying the housing design and enhancing the housing appearance.

## Claims

1. A portable electronic device, comprising:
a sensing module comprising a button, a transmitter unit, and a receiver unit, wherein the sensing module transmits at least one trigger signal when the button is pressed, the transmitter unit is disposed inside the button, and the transmitter unit transmits a first optical signal towards an outside of the button through the button, and the receiver unit is disposed inside the button, the receiver unit receiving a second optical signal through the button, wherein the second optical signal is an optical signal that is reflected when the first optical signal encounters an object; and
a transceiver circuit coupled to the sensing module, controlling the transmitter unit to transmit the first optical signal towards the outside of the button through the button in response to the trigger signal, and the transceiver circuit obtains physiological information according to the second optical signal, wherein the button has a transparent material to allow the first optical signal and the second optical signal to travel through the button.

2. The portable electronic device according to claim 1, wherein the sensing module further comprises:
a sensor, disposed inside the button and coupled to the transceiver circuit, sensing whether the button is pressed and transmitting the trigger signal when the button is pressed.

3. The portable electronic device according to claim 1, wherein
the first optical signal is an infrared signal or a red light signal;
the transmitter unit further comprises an optical transmitter transmitting the first optical signal through the button; and
the receiver unit further comprises an optical receiver receiving the second optical signal through the button.

4. The portable electronic device according to claim 3, wherein the optical transmitter is further controlled by the transceiver circuit to transmit a third optical signal towards the outside of the button through the button so as to remotely control another electronic device, and the optical receiver receives a fourth optical signal from a remote control through the button.

5. The portable electronic device according to claim 1, wherein
the first optical signal comprises an infrared signal and a red light signal;
the transmitter unit further comprises a first optical transmitter and a second optical transmitter, the first optical transmitter transmits the infrared signal through the button, and the second optical transmitter transmits the red light signal through the button; and
the receiver unit further comprises an optical receiver receiving the reflected infrared signal and the reflected red light signal through the button.

6. The portable electronic device according to claim 1, wherein
the first optical signal comprises an infrared signal and a red light signal;
the transmitter unit further comprises a first optical transmitter and a second optical transmitter, the first optical transmitter transmits the infrared signal through the button, and the second optical transmitter transmits the red light signal through the button; and
the receiver unit further comprises a first optical receiver and a second optical receiver, the first optical receiver receives the reflected infrared signal through the button, and the second optical receiver receives the reflected red light signal through the button.

7. The portable electronic device according to claim 1, wherein the transceiver circuit comprises:
a controller coupled to the sensing module and transmitting a first electric signal according to the trigger signal;
a first amplifier coupled between the controller and the sensing module, the first amplifier amplifying the first electric signal, wherein the transmitter unit converts the amplified first electric signal into the first optical signal, and the receiver unit converts the second optical signal into a second electric signal;
a filter coupled to the receiver unit and filtering out a noise signal in the second electric signal;
a second amplifier coupled to the filter and amplifying the second electric signal;
an analog-to-digital converter coupled to the second amplifier and converting the amplified second electric signal from an analog signal to a digital signal; and
a storage unit coupled to the analog-to-digital converter and the controller, the storage unit storing a binary value corresponding to the digital signal, wherein the controller obtains the physiological information according to the binary value.

8. The portable electronic device according to claim 1, wherein the controller further transmits a first remote control command, and the transceiver circuit further comprises:
a modulator coupled to the controller and modulating and generating a second electric signal according to the first remote control command; and
a second amplifier coupled between the modulator and the sensing module, the second amplifier amplifying the second electric signal, wherein the transmitter unit converts the amplified second electric signal into a third optical signal, and the transmitter unit transmits the third optical signal towards the outside of the button through the button, so as to remotely control another electronic device.

9. The portable electronic device according to claim 8, wherein the receiver unit further receives a fourth optical signal through the button, and the receiver unit converts the fourth optical signal into a third electric signal, and the transceiver circuit further comprises:
a filter coupled to the receiver unit and filtering out a noise signal in the third electric signal;
a third amplifier coupled to the filter and amplifying the third electric signal; and
a demodulator coupled to the third amplifier and demodulating the amplified third electric signal to obtain a second remote control command.

10. The portable electronic device according to claim 1, wherein the physiological information at least comprises one of a heart beat rate, a heart waveform, and a blood oxygenation level.

11. The portable electronic device according to claim 1, wherein the button is a power button, and the portable electronic device further comprises:
a display; and
a processor coupled to the sensing module and the display, the processor controlling the portable electronic device to enter an operating state from a sleep state according to the trigger signal, wherein the display is turned off in the sleep state, and the display is turned on in the operating state so as to display an operating image.

12. The portable electronic device according to claim 11, further comprising:
a storage device coupled to the processor, wherein the transceiver circuit transmits a notification signal to the processor, the processor obtains the physiological information from the transceiver circuit according to the notification signal, and the processor stores the physiological information in the storage device.

13. A method for physiological measurement, executed by a portable electronic device, the portable electronic device comprising a button and a display, the method comprising:
transmitting at least one trigger signal when the button is pressed;
transmitting a first optical signal towards an outside of the button through the button according to the trigger signal;
receiving a second optical signal through the button, wherein the second optical signal is an optical signal that is reflected when the first optical signal encounters an object; and
obtaining physiological information according to the second optical signal.

14. The method according to claim 13, wherein the physiological information at least comprises one of a heart beat rate, a heart waveform, and a blood oxygenation level.

15. The method according to claim 13, further comprising:
comparing the physiological information with another physiological information stored previously; and
when the physiological information matches the another physiological information, unlocking a locked state of the display.
